# EUROPEAN PATENT SPECIFICATION

(11) **EP 0 893 137 B1**
(45) Date of publication and mention of the grant of the patent: **31.03.2004**
(21) Application number: 98304054.4
(22) Date of filing: 21.05.1998
(51) Int. Cl.: A61M 25/06, A61M 5/32

(54) **Assembly for an indwelling catheter and method of making it**
Verweilkathetersatz und Herstellungsmetode
Assemblage de cathéter à demeure et méthode pour sa fabrication

(30) Priority: 22.07.1997 JP 21134697
(43) Date of publication of application: 27.01.1999
(73) Proprietor: TERUMO KABUSHIKI KAISHA, Tokyo 151-0072 (JP)
(72) Inventor: Nakada, Tsuneo, Nakakoma-gun Yamanashi (JP); Kobayashi, Masahiko, Nakakoma-gun Yamanashi (JP)
(74) Representative: Harrison, David Christopher

(56) References cited:
- WO-A-93/13821
- US-A- 4 377 165
- US-A- 5 279 572

## Description

For an infusion into a patient, an indwelling catheter, connected with an infusion line, is inserted and remains in a blood vessel of the patient.

The indwelling catheter is inserted into the blood vessel by fitting a hollow needle inside the catheter so that a sloped leading end of the needle point protrudes from the leading end of the catheter, to form a catheter assembly.

When the inner needle reaches the inside of the blood vessel, the blood coming from the open needle point flows (or "flashes back") into a transparent housing which is mounted at the root portion of the needle. The operator thus can confirm visually that the needle has captured the blood vessel.

The needle and the catheter are then slightly moved forward, to insert the leading end of the catheter into the blood vessel.

In most indwelling catheter assemblies of the prior art, however, the inner circumference of the leading end of the catheter is closely fitted all around the outer circumference of the needle so that the blood will not flow between the outer catheter and the needle. This makes it impossible to confirm visually by blood flashback whether or not the catheter has captured the blood vessel.

After confirmation of the capture of the blood vessel by the needle, therefore, the needle and the catheter are delicately advanced according to the feel and experience of the operator. Proper insertion of the catheter into the blood vessel is not always ensured; it may be too shallow or too deep, partly because the spacing between the edge face of the needle and the leading end of the catheter is slightly different depending upon the kind, size and maker of the indwelling catheter assembly and partly because the size of blood vessels of patients is different.

It has been proposed in US-A-5279572 which is considered as the closest prior art, to machine an axial groove in the outer surface of the needle so that blood from the blood vessel can pass the area where the catheter fits tightly around the needle and allow a visual indication to the operator that the catheter itself has entered the blood vessel. The needle can then be withdrawn leaving the catheter in a position not solely determined by the experience and skill of the operator, and correct for that particular patient.

It is however a disadvantage of that proposal that the groove is sharp-edged, and is formed by machining into the wall of the needle, which in a typical case can have an initial radial thickness of only some 0.1 to 0.2 mm.

Japanese Utility Model U-3-85046 shows an inner needle which has a sharp-edged groove in an otherwise uniform-thickness wall.

Any thinning in a uniform such wall leads to its being weakened. In the US patent the inventor provided a thickened portion into which the groove is cut. This is wasteful of material and complex to manufacture.

Sharp edges where the groove intersects the outer surface of the needle can cause pain to the patient or cause shaving of the catheter material during relative movement; the latter is particularly undesirable. The sharp edges could only be removed by a further processing step.

An object of the invention is to provide an indwelling catheter assembly which while allowing visual confirmation that the indwelling catheter has captured a blood vessel is easily manufactured and avoids the disadvantages of the prior art.

Accordingly, in a first aspect there is provided a catheter assembly as defined in claim 5 herein. This assembly uses a grooved needle of which, in a single manufacturing step, the groove is formed to meet the outer surface of the remainder of the needle at a smooth edge.

The grooved needle has a substantially uniform wall thickness between outer and inner surfaces in and around the groove.

The groove is one which has been formed by press-working.

Normally the needle will be metallic and will be of uniform radial wall thickness.

The invention also provides a method of making a needle for an assembly for an indwelling catheter as defined in claim 1 herein. The method includes forming an axially-extending groove along at least part of a tube by press-working the material of the tube while leaving a lumen open under the groove.

Normally, press-working will be effected after formation of the inclined face of the needle and the groove will intersect that face where it is furthest from the extreme tip formed by that face. However, the inclined end face may be formed after the formation of the groove.

Of course, press-working is *per se* a known process. It has also be proposed for use in the context of medical needles, by Japanese Patent Application A-7-32803. However, there it was used for the complete end-closure of a side-ported syringe needle.

That press-working has been used on a given needle is clearly evident to a man skilled in the art. If a groove is formed by press-working of a cylindrical needle tube after the inclined end-face has been formed and the groove intersects that end face there will be an indentation into the plane of inclination where the groove departs from the cylindricality of the tube. Press-working is also detectable by polishing and etching a cross-section through a metallic needle; disruption of the structure at the zones of stress will be evident on microscopic examination.

It is preferable that the groove intersects the inclined end face since the detection of entry of the catheter into the blood vessel will occur at the earliest possible moment.

It is highly preferable that a tube of uniform wall-thickness and cylindrical shape is used for the process, since this is simplest and cheapest; press-working results in no substantial change in wall-thickness and in and around the groove, and results in a smooth-edged groove.

One embodiment of indwelling catheter assembly and method of the invention will now be described in detail with reference to the accompanying drawings.
Figure 1 is a longitudinal section showing the embodiment of the indwelling catheter assembly of the invention;
Figure 2 is a longitudinal section showing the vicinity of the leading end when the inner needle and the outer catheter are assembled;
Figure 3 is a top plan view showing the vicinity of the leading end of the inner needle of the indwelling catheter assembly shown in Figure 1;
Figure 4 is a section taken along line IV - IV of Figure 3;
Figure 5 is a section taken along line V - V of Figure 3;
Figure 6 is a diametrical section through the leading end of the catheter assembly;
Figure 7 is a diagrammatic perspective view of a press-working machine;
Figure 8 is a partial vertical section on the plane VIII - VIII of Figure 7;
Figure 9 is a partial vertical section on the plane IX - IX of Figure 7;
Figure 10 is a partial vertical section on the plane IX - IX of Figure 7 with the pressing tool closed; and
Figure 11 is a photomicrograph of a cross-sectioned needle embodying the invention.

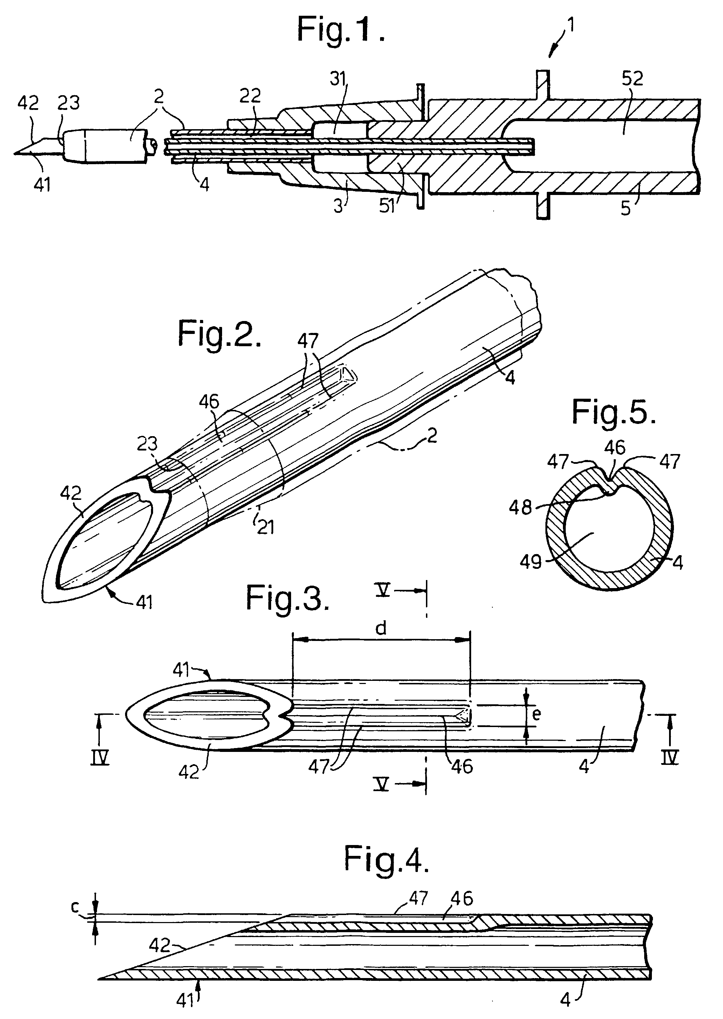

In the following description, the right hand side of Figures 1 to 4 will be called the "root end", whereas the left hand side will be called the "leading end".

As shown in Figure 1, an indwelling catheter assembly 1 includes a catheter 2 and a hollow inner needle 4.

On the root end of the outer catheter 2 there is a liquid-tight fixed hub 3. This hub 3 is made of a transparent or semitransparent resin to enable visibility of its chamber 31.

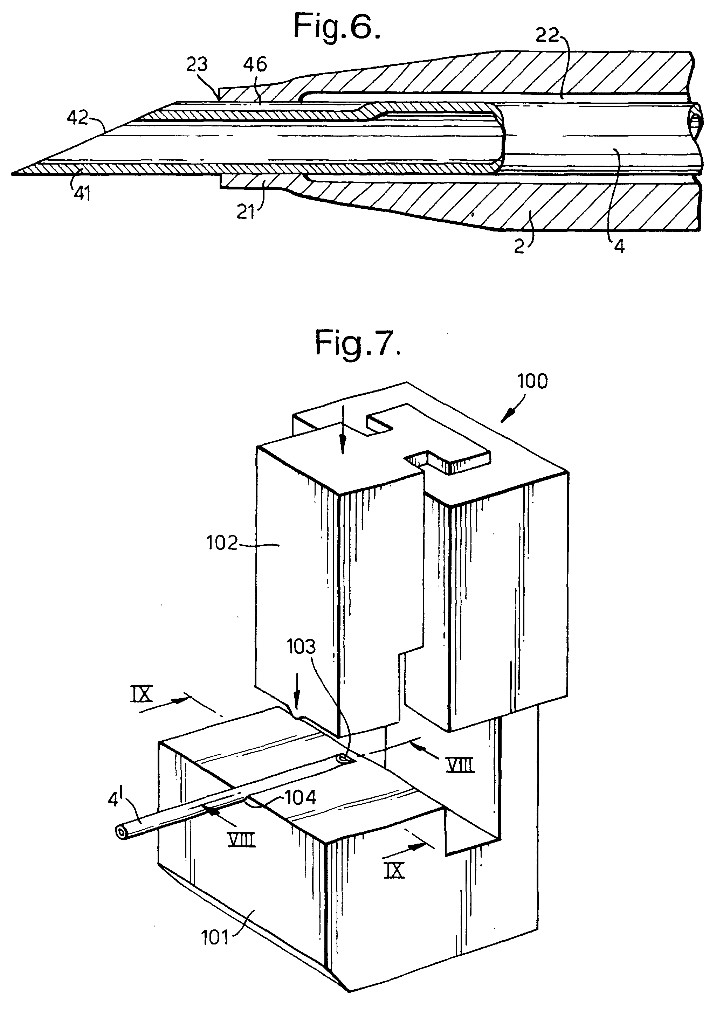

As shown in Figure 6, the leading end of the catheter 2 has a constricted portion 21. The internal diameter of this constricted portion 21 is made substantially equal to or slightly smaller than the external diameter of the needle 4 so that the inner circumference of the constricted portion 21 comes into close contact with the outer circumference of the needle 4 when the latter is inserted into the outer catheter 2 to the extent that its needle point 41 protrudes from the leading end opening 23 of the catheter 2 (this will be called the "assembled state") - see Figures 1 and 6.

In the portion closer to the root end than the constricted portion 21 the catheter 2 has a larger internal diameter than the external diameter of the needle 4 so that a predetermined clearance 22 is formed in the assembled state between the inner circumference of the constricted portion 21 and the outer circumference of the needle 4. This clearance 22 provides a passage for blood.

The leading end of the catheter 2 is externally tapered, with its external diameter gradually decreasing toward the leading tip. As a result, it may be inserted into an organ easily and with low trauma.

Any suitable material may be used for the catheter 2; preferable examples are soft resins such as ethylene tetrafluoroethylene (ETFE), polyurethane or polyether nylon resin.

This catheter 2 is preferably transparent or translucent so as to allow visibility over the whole or part of it. Into the material of the catheter 2, moreover, there can be blended an x-ray contrast medium such as barium sulfate or barium carbonate to provide a contrast function.

The needle 4 will usually be made of a metallic material such as stainless steel, aluminium or an alloy thereof, or titanium or an alloy thereof and has the sharp needle point 41 at its leading end. This needle point 41 has an end face 42 which is inclined at a predetermined angle with respect to the axis of the needle 4.

On the root end of the needle 4, there is a liquid-tight fixed hub 5. This hub 5 is preferably made of a transparent or semitransparent resin to allow visibility to its inside chamber 52.

At the leading end of the hub 5, there is formed a spigot 51, which is held in the root end opening of the catheter hub 3 in the assembled state.

Suitable materials for making the catheter hub 3 and the needle hub 5 respectively are polyolefins such as polyethylene, polypropylene or a copolymer of ethylene and vinyl acetate, polyvinyl chloride, polymethyl methacrylate, polycarbonate, polybutadiene, polyamide or polyester.

Figures 2 to 5 show the construction of the leading end of the needle 4.

In the outer circumference of the leading end of the needle 4, there is formed a groove 46 which extends along the longitudinal (axial) direction of the needle 4. A lumen 49 remains open under the bottom 48 of the groove and communicates with the tip 41 of the needle.

In the assembled state, as shown in Figure 6, the groove 46 is positioned to confront the constricted portion 21 of the outer catheter 2 and to extend axially beyond the constricted portion 21.

In this case, as shown in Figures 3, 5 and 11, the two edges 47 of the groove 46, i.e. the vicinities of the boundary between the groove 46 and remainder of the outer circumference of the needle 4 are rounded and smooth.

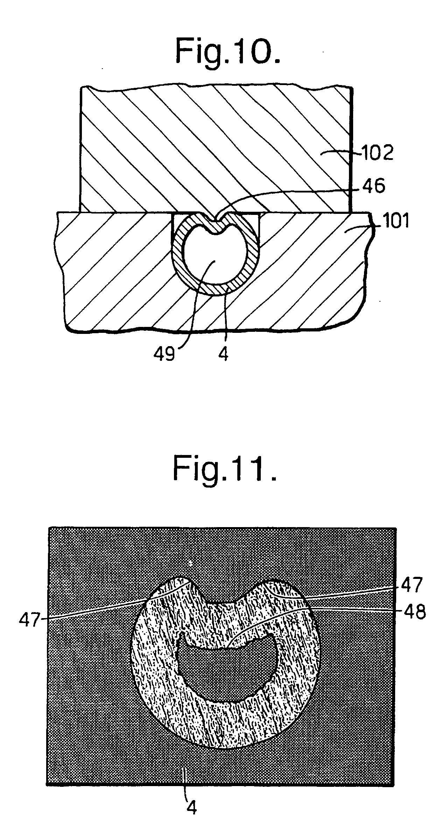

When the needle 4 is elastically deformed by press-working to form the groove 46, this groove 46 can be easily given the rounded edges 47 without any secondary working. Considering that the inner needle usually has a radial wall thickness of about 0.1 to 0.2 mm, it is a great strength advantage that the wall thickness remains as it was and is constant in and around the groove, as can most clearly be seen from Figure 11. This constancy includes possible minor drawing and compressive changes in the thickness due to the pressing.

The press-working can be performed, for example, by an apparatus 100 shown in Figure 7.

A tube 4' which will be the needle 4, having the inclined face 42 formed in advance, is fitted in a U-shaped groove 104 of a female mould 101. This U-shaped groove 104 has a diameter substantially equal to or only slightly larger than the external diameter of the needle 4 so that it will not be transversely crushed when pressed. Since this groove is to be formed along the axial direction from the root portion of the edge face i.e. the portion of that face most remote from the extreme tip of the needle, the position of the tube has to be accurately adjusted.

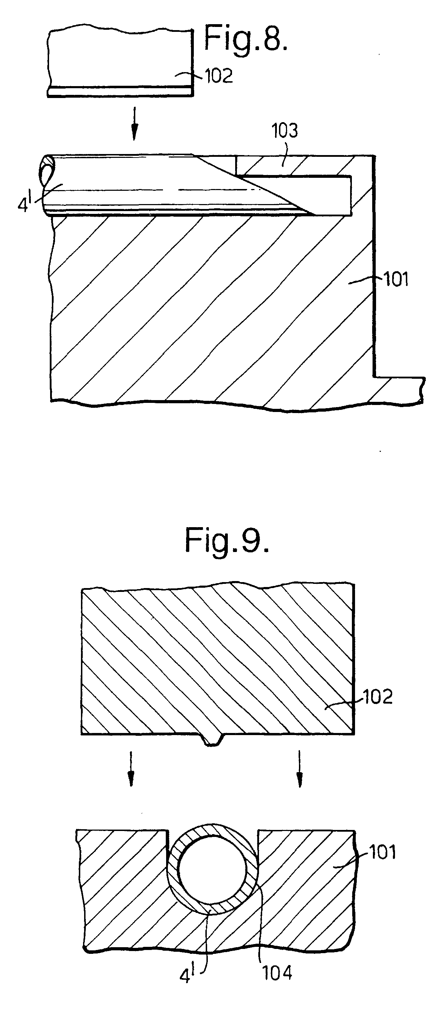

Figure 8 shows on a larger scale the point portion 41 of the needle 4 in the female mould 101. As shown, the inclined face 42 of the inner needle 4 is in abutment against a planar overhanging ledge 103. With this arrangement, the inclined face can easily be orientated correctly.

Although not shown, the position at which the press tool will act can be adjusted by checking the orientation of the inner needle by cameras.

After this adjustment of the edge face, the tube is pressed by a male tool 102 (as shown in Figures 9 and 10). Depending on the material, diameter and thickness of the tube forming the needle, the pressing pressure may be 50 to 300 Kg/cm², preferably 100 to 300 Kg/cm².

The shape and size of the groove 46 is not critical except the groove 46 must in the assembled state go beyond the contacting portion 21 between the needle 4 and the catheter 2. In the shown construction, however, the groove 46 preferably has a maximum depth c of about 0.01 to 0.3 mm, more preferably about 0.03 to 0.15 mm, and preferably has a length d of about 1 to 30 mm, more preferably about 5 to 15 mm. Moreover, the groove 46 preferably has a width e of about 0.1 to 1.0 mm, more preferably about 0.2 to 0.5 mm. The outer diameter of a cylindrical tube forming the needle will usually be in the order of 0.4 to 1.7 mm, with the tube having a uniform radial wall thickness of 0.1 to 0.2 mm.

The sectional shape, maximum depth and width of the groove 46 may be constant or be varied along the longitudinal direction of the inner needle 4. For example, the groove 46 may have a portion (especially at its root end portion), where the maximum depth and/or width are gradually decreased toward the root end.

According to the aforementioned press-working, it is possible to form easily plural grooves 46 or groove 46 of a complicated shape. Furthermore, by using appropriate moulds, grooves 46 can be formed respectively in plural needles in a single operation.

In summary, as has been described hereinbefore, a blood passage is formed by press-working the outer circumference of the needle so that the capture of the blood vessel by the catheter can be confirmed in terms of the flashback of the blood. As a result, the insertion of the indwelling catheter into the blood vessel can be performed easily and accurately.

Moreover, the indwelling catheter assembly can be easily manufactured, with high accuracy.

## Claims

1. A method of making a catheter assembly of an indwelling catheter, the assembly having a hollow catheter (2) surrounding a hollow needle (4) and having a portion (21) tightly engaging the needle, the needle having a groove (46) for permitting passage of blood past the tightly-engaging portion of the catheter while leaving a lumen (49) within the needle (4),
**characterized in that** the method includes forming the groove (46) by press-working the needle tube (4'), the press-working resulting in no substantial change in wall thickness in and around the groove and **in that** the groove has smooth transitions (47) between the groove and the remainder of the outer surface of the needle tube (4').

2. A method according to claim 1, wherein press-working is carried out on a needle tube (4') which is a cylinder of uniform radial wall thickness.

3. A method according to claim 1 or claim 2, wherein the method includes, before forming the groove, forming an inclined end face (42) to provide a sharp tip (41) of the needle, and forming the groove (46) to intersect with that inclined face where it is most remote from the sharp tip.

4. A method according to claim 3, further including placing the tube (4') on a female mould (101) of a press-working machine and causing the inclined end face (42) to abut with a surface (103) at an end of the female mould (101) to define the orientation of the needle tube relative to a male tool (102) of the machine.

5. A catheter assembly of an indwelling catheter, the assembly having a hollow catheter (2) surrounding a hollow needle (4) and having a portion (21) tightly engaging the needle, the needle having a groove (46) for permitting passage of blood past the tightly-engaging portion of the catheter while leaving a lumen (49) within the needle (4),
**characterized in that** the wall thickness in and around the groove (46) is substantially constant and **in that** the groove (46) has smooth transitions (47) to the remainder of the outer surface of the needle (4), as seen in cross-section.

6. An assembly according to claim 5, wherein the needle (4) is of constant cross-sectional thickness.

7. An assembly according to claim 5 or claim 6, wherein the needle (4) is cylindrical but for the groove (46).

8. An assembly according to any one of claims 5 to 7, wherein the needle (4) has an inclined end face (42) to provide a sharp tip (41) of the needle, and the groove (46) intersects with that inclined face where it is most remote from the sharp tip.

## Patentansprüche

1. Verfahren zur Herstellung einer Katheteranordnung eines Dauerkatheters, wobei die Anordnung einen hohlen Katheter (2) aufweist, der eine Hohlnadel (4) umgibt und einen Abschnitt (21) aufweist, der sich in dichtendem Eingriff mit der Nadel befindet, wobei die Nadel eine Rille (46) aufweist, um das Hindurchtreten von Blut vorbei am Abschnitt mit dichtendem Eingriff des Katheters zuzulassen, während eine lichte Weite (49) innerhalb der Nadel (4) verbleibt,
**dadurch gekennzeichnet, dass** das Verfahren das Ausbilden der Rille (46) durch Pressformen der Nadelröhre (4') umfasst, wobei das Pressformen zu keiner wesentlichen Veränderung der Wanddicke in und um die Rille führt, und dass die Rille glatte Übergänge (47) zwischen der Rille und dem Rest der Außenfläche der Nadelröhre (4') aufweist.

2. Verfahren nach Anspruch 1, worin das Pressformen an einer Nadelröhre (4') vorgenommen wird, die ein Zylinder mit gleichmäßiger radialer Wanddicke ist.

3. Verfahren nach Anspruch 1 oder 2, worin das Verfahren vor dem Ausbilden der Rille das Ausbilden einer geneigten Endfläche (42), um eine scharfe Spitze (41) der Nadel bereitzustellen, und das Ausbilden der Rille (46) auf solche Weise umfasst, das sie diese geneigte Fläche dort schneidet, wo sie von der scharfen Spitze am weitesten entfernt ist.

4. Verfahren nach Anspruch 3, weiters umfassend das Anordnen der Röhre (4') auf einer Matrize (101) einer Pressformungsmaschine und das Anliegen-Lassen der geneigten Endfläche (42) an eine Oberfläche (103) an einem Ende der Matrize (101), um die Ausrichtung der Nadelröhre in Bezug auf eine Patrize (102) der Maschine zu definieren.

5. Katheteranordnung eines Dauerkatheters, wobei die Anordnung einen hohlen Katheter (2) aufweist, der eine Hohlnadel (4) umgibt und einen Abschnitt (21) aufweist, der sich in dichtendem Eingriff mit der Nadel befindet, wobei die Nadel eine Rille (46) aufweist, um das Hindurchtreten von Blut vorbei am Abschnitt mit dichtendem Eingriff des Katheters zuzulassen, während eine lichte Weite (49) innerhalb der Nadel (4) verbleibt,
**dadurch gekennzeichnet, dass** - im Querschnitt betrachtet - die Wanddicke in und um die Rille (46) im Wesentlichen konstant ist und dass die Rille (46) glatte Übergänge (47) zum Rest der Außenfläche der Nadel (4), aufweist.

6. Anordnung nach Anspruch 5, worin die Nadel (4) eine konstante Querschnittsdicke aufweist.

7. Anordnung nach Anspruch 5 oder 6, worin die Nadel (4) mit Ausnahme der Rille (46) zylindrisch ist.

8. Anordnung nach einem der Ansprüche 5 bis 7, worin die Nadel (4) eine geneigte Endfläche (42) aufweist, um eine scharfe Spitze (41) der Nadel bereitzustellen, und die Rille (46) die geneigte Fläche dort schneidet, wo sie am weitesten von der scharfen Spitze entfernt ist.

## Revendications

1. Procédé de fabrication d'un ensemble formant cathéter d'un cathéter à demeure, l'ensemble comportant un cathéter creux (2) entourant une aiguille creuse (4) et présentant une portion (21) en prise étanche avec l'aiguille, l'aiguille présentant une rainure (46) pour permettre le passage du sang le long de la portion hermétiquement étanche du cathéter tout en laissant subsister une lumière (49) dans l'aiguille (4),
**caractérisé en ce que** le procédé comprend la formation de la rainure (46) par un travail de pressage du tube d'aiguille (4'), le travail de pressage n'entraînant pas de changement important de l'épaisseur de paroi dans et autour de la rainure, et **en ce que** la rainure a des transitions régulières (47) entre la rainure et le reste de la surface externe du tube d'aiguille (4').

2. Procédé selon la revendication 1, où le travail de pressage est exécuté sur un tube d'aiguille (4') qui est un cylindre d'une épaisseur de paroi radiale uniforme.

3. Procédé selon la revendication 1 ou la revendication 2, où le procédé comprend, avant la formation de la rainure, la formation d'une face d'extrémité inclinée (42) pour réaliser une pointe tranchante (41) de l'aiguille, et la réalisation de la rainure (46) pour qu'elle se croise avec cette face inclinée qui est la plus éloignée de la pointe tranchante.

4. Procédé selon la revendication 3, comprenant en outre les étapes consistant à placer le tube (4') sur un moule femelle (101) d'une machine de pressage et à amener la face d'extrémité inclinée (42) à buter contre une surface (103) à l'extrémité du moule femelle (101) pour définir l'orientation du tube d'aiguille relativement à un outil mâle (102) de la machine.

5. Ensemble formant cathéter d'un cathéter à demeure, l'ensemble présentant un cathéter creux (2) entourant une aiguille creuse (4) et présentant une portion (21) en prise étanche avec l'aiguille, l'aiguille présentant une rainure (46) pour permettre le passage du sang au-delà de la portion fortement en prise du cathéter tout en laissant subsister une lumière (49) dans l'aiguille (4),
**caractérisé en ce que** l'épaisseur de paroi dans et autour de la rainure (46) est sensiblement constante, et **en ce que** la rainure (46) a des transitions régulières (47) vers le restant de la surface externe de l'aiguille (4), vue en section transversale.

6. Ensemble selon la revendication 5, où l'aiguille (4) a une épaisseur constante en section transversale.

7. Ensemble selon la revendication 5 ou la revendication 6, où l'aiguille (4) est cylindrique à l'exception de la rainure (46).

8. Ensemble selon l'une des revendications 5 à 7, où l'aiguille (4) possède une face d'extrémité inclinée (42) pour réaliser une pointe tranchante (41) de l'aiguille, et la rainure (46) se croise avec cette face inclinée à l'endroit le plus éloigné de la pointe tranchante.
